# EUROPEAN PATENT APPLICATION

(11) **EP 3 620 157 A1**
(43) Date of publication of application: **11.03.2020**
(21) Application number: 19190193.3
(22) Date of filing: 02.05.2002
(51) Int. Cl.: A61K 9/51, A61K 31/436, A61L 29/08, A61L 29/16, A61L 31/10, A61L 31/16, B82Y 5/00, A61P 35/00, A61K 31/335, A61K 31/337, A61K 31/495, A61K 31/427, A61K 38/13, A61K 45/06

(54) **COMPOSITION FOR USE IN A METHOD FOR TREATING HYPERPLASIA**

(30) Priority: 02.05.2001 US 847945
(62) Divisional of application: 02731657.9
(71) Applicant: Abraxis BioScience, LLC, Summit, NJ 07901 (US)
(72) Inventor: DESAI, Neil P., Pacific Palisades, CA California 90272 (US); SOON-SHIONG, Patrick, Culver City, CA California 90232 (US)
(74) Representative: Jones Day

(57) **Abstract**

Provided herein is a pharmaceutical composition comprising nanoparticles comprising rapamycin and human serum albumin, as well as the use of the composition in a method of treating hyperplasia in a subject in need thereof.

## Description

### RELATED APPLICATIONS

This application is a continuation-in-part of United States Application Serial No. 09/446,783, filed May 16, 2000, now pending, which in turn claims priority from PCT Application No. US98/13272, which, in turn, claims priority from United States Application No. 60/051,021, filed June 27, 1997, each of which is hereby incorporated by reference herein in its entirety.

### FIELD OF THE INVENTION

The present invention relates to methods for the treatment of hyperplasia and compositions useful therefor.

### BACKGROUND OF THE INVENTION

Coronary atherosclerosis is caused by fatty deposits called plaque that narrow the cross section available for blood flow through the coronary arteries, which supply blood to the muscle of the heart. To treat patients with this condition, cardiac surgeons often use a procedure called coronary artery bypass grafting (CABG). Typically, the saphenous vein is harvested from the patient's leg, trimmed to size, and grafted to the artery, thus bypassing the blockage. Although generally effective, the procedure carries risks ranging from infection to death and usually involves painful closure wounds.

Under certain circumstances, interventional cardiologists choose to treat the blockage rather than bypass it, using a minimally invasive technique called percutaneous transluminal coronary angioplasty (PTCA). In PTCA, a catheter is typically inserted through the femoral artery in the patient's leg, threaded into the blocked coronary artery, and inflated. The plaque is compressed into the vessel wall and the lumen or flow cross section of the artery is thus enlarged. A less common technique called directional coronary atherectomy (DCA) can be used in conjunction with or instead of PTCA to literally cut plaque from the wall. To treat calcified coronary arteries, a related technique called rotational coronary atherectomy (RCA) can be employed to remove calcified plaque with a high-speed rotating burr. Unfortunately, the body's response to these procedures often includes thrombosis or blood clotting and the formation of scar tissue or other trauma-induced tissue reactions-for example, at the PTCA site. Statistics show that restenosis or renarrowing of the artery by scar tissue occurs in fully one-half of the treated patients within only 6 months after these procedures.¹ Restenosis in injured blood vessels as a result of angioplasty, atherectomy or the placement of a stent is the result of the normal healing response which involves proliferation of smooth muscle cells as well as migration of smooth muscle cells into the area of vascular injury. Paclitaxel has been demonstrated to prevent or minimize the degree of restenosis by reducing migration and proliferation of vascular smooth muscle cells.

To prevent restenosis, cardiologists often place a small metal tubular device called an intracoronary stent at the PTCA site. Stents are scaffolding devices that maintain vessel patency after an interventional procedure, usually balloon angioplasty. Stents provide mechanical scaffolding that reduces early elastic recoil or dissection and eliminates late lumen loss by circumferential remodeling.^{2,3} Coronary stenting is now used in more than 50% of patients undergoing nonsurgical myocardial revascularization.⁴ It is considered a routine adjunct to coronary angioplasty. In 1998, coronary stents were placed in an estimated 500,000 patients in the United States, with an average of 1.7 stents inserted per patient.⁵

Results of several clinical studies suggest that the rate of restenosis is significantly reduced in certain indications by the use of coronary stents. Among the first published studies, the Benestent and Stent Restenosis Study (STRESS) trials reported restenosis rates of 33% and 25%, respectively, with coronary stenting.⁶ A subsequent study reported that 11% of patients with acute myocardial infarction who received stents experienced restenosis, compared with 34% in the PTCA-only group.7

Stents, however, are not free of complications. Although aggressive antiplatelet therapy has minimized early stent thrombosis, in-stent restenosis represents the most important drawback to stenting. Restenosis occurs because of neointimal proliferation of cells through the latticework of the stent. This occurs to some extent in all patients, but in most the process stops before the artery is occluded. Restenosis occurs in those patients who have an overexuberant growth of scar tissue. In general, another interventional coronary procedure is required.

Paclitaxel (taxol), a potent antineoplastic drug, is approved for the therapy of ovarian, breast, and other cancers.⁸ Two preliminary studies have investigated the use of paclitaxel to reduce in-stent restenosis in *porcine coronary arteries.*^{9,10} Stents coated with a biodegradable polymer containing slow-release paclitaxel (175-200µg/stent estimated to be released at a rate of 0.75µg/day) was associated with a reduction in diameter stenosis and neointimal area at 4 weeks. It is unknown whether local pathological effects were present. In another study,¹⁰ paclitaxel was directly applied to stents (without a biodegradable polymer) and deployed in the coronary arteries. Lumen area was increased with 15 and 90 µg paclitaxel stents, and there was a significant reduction in neointimal area with 90 µg paclitaxel stents. However, significant local cytotoxic effects were observed in stents coated with 90 µg of paclitaxel.

Although local paclitaxel delivery via stents is attractive and clinical trials in humans are presently underway in Europe, the enthusiasm for this approach is tempered by a possible delaying of arterial healing. Furthermore, the potential toxic effects of locally administered paclitaxel are augmented by the presence of a stent acting as a local foreign body. Finally, the in vivo intra-arterial release kinetics of paclitaxel from a coated stent over time is unknown.

The market for treatment of coronary restenosis is linked with the market for coronary stents. The coronary stent market is among the fastest growing U.S. medical device markets. Different reports cite varying numbers for the yearly total for implanted stents. The following excerpts give a general perspective of the stent market that appears to total between 500,000 to 1,000,000 units annually.
"More than 20% of the estimated one million stents implanted annually develop blockages, which can lead to partial or total obstruction of the stented artery." (Nov. 16, 1999, PRNewswire, The Spectranetics Corporation Press release)
"More than 700,000 angioplasties take place in the United States each year and physicians consider the use of stents in a large percentage of these cases when vessels threaten to reclose." (Oct. 28; 1999, PRNewswire, Medtronic, Inc. Press release)
"Coronary stenting is now used in more than 50% of patients undergoing nonsurgical myocardial revascularization.1 It is considered a routine adjunct to coronary angioplasty. In 1998, coronary stents were placed in an estimated 500,000 patients in the United States, with an average of 1.7 stents inserted per patient." (The Growing Role of Stents in Coronary Disease, The Medical Journal of Allina, Vol 8, No. 3, Summer 1999)

Although stents are used most often in coronary arteries; they are also used in other vessels. Those most often chosen are the carotid, abdominal, and renal arteries. Stent placement in the carotid artery may eventually become an alternative to surgical endartercotmy. At present, however, the American Heart Association has recommended that carotid artery stenting be performed only within clinical trial settings. No established techniques or guidelines exist. Stent placement in the abdominal aorta may be used as an alternative to major surgery whereby aneurysms in the vassel can be sealed off with covered stents. Stenting is also the procedure of choice in renal artery. Surgery in this case is not a good alternative. It has been shown that patients with stented renal arteries have a reduction in the need for hypertension medication and dialysis, as well as a lower risk of renal failure. There is also a growing need for "peripheral" stents and each year in the US, 70% of the 160,000 hemodialysis patients requires access to the circulatory system for ongoing medical treatment. Unfortunately, passageway narrowing is a significant problem, representing yet an additional need for an effective therapy for reduction or prevention of stenosis in these blood vessels.

### Background References

1. S Goldberg et al., "Coronary Artery Stents," Lancet 345 (1995): 1523-1524.
2. Serruys PW, De Jaegere P, Kiemeneij F, et al, for the Benestent Study Group. A comparison of balloon expandable stent implantation with balloon angioplasty in patients with coronary artery disease. N Engl J Med. 1994; 331:489-495.
3. Fischman DL, Leon MB, Baim DS, et al, for the Stent Restenosis Study Investigators. A randomized comparison of coronary-stent placement and balloon angioplasty in the treatment of coronary artery disease. N Engl J Med. 1994; 331:496-501.
4. Holmes DR Jr. Hirshfeld J Jr. Faxon D, et al ACC Expert Consensus document on coronary artery stents: document of the American College of Cardiology. J Am Coll Cardiol. 1998;32:1471-1482.
5. Topol EJ Coronary artery stents -- gauging. gorging, and gouging. N Engl J Med. 1998;339:1702-1704.
6. S Goldberg et al., "A Meta-Analysis on the Clinical and Angiographic Outcomes of Stents vs. PTCA in the Different Coronary Vessels in the Benestent-I and STRESS-1 and 2 Trials," Journal of the American College of Cardiology 27, no. 2 (1996): supp. A 80A.
7. H Suryapranata et al., "Randomized Comparison of Coronary Stenting with Balloon Angioplasty in Selected Patients with Acute Myocardial Infarction," Circulation 97 (1998): 2502-2505.
8. Gelmon K. The taxoids: paclitaxel and docetaxel. Lancet. 1994;344:1267-1272.
9. Kornowski R, Hong MK, Ragheb AO, Leon MB. Slow release taxol coated GR11 stents reduce neointima formation in a coronary in-stent restenosis model. Circulation 1997;96 (supplement I):I-341.
10. Heldman AH, Cheng L, Heller P, Jenkins Gm, Ware M, Nater C, Rezai B, Hruban RH, Sollott SJ, kinsella J, Lakatta EG, Brinker JA, Froehlich j. Paclitaxel applied directly to stents inhibits neointimal growth without thrombotic complications in a porcine coronary artery model of restenosis. Circulation 1997;96: (supplement I):I-288.

### OBJECTS OF THE INVENTION

It is, therefor, an object of the invention to identify formulations useful in conjunction with devices such as catheters, stents, and the like, to facilitate the treatment of subjects in need thereof.

It is another object of the present invention to identify formulations useful for administration of suitable drugs in conjunction with procedures such as balloon angioplasty or stenting to significantly reduce the level of restenosis.

It is yet another object of the present invention to identify formulations useful for administration of suitable drugs to a subject in need thereof, either before, during or after a procedure such as angioplasty of stenting to reduce the level of restensosis in such subjects.

It is still another object of the present invention to identify formulations useful for administration of suitable drugs to a subject in need thereof at desirable intervals following a procedure such as angioplasty or stenting to reduce the level of restensosis in such subjects.

It is a further object of the present invention to identify formulations useful for administration of one or more drugs to a subject in need thereof either before, during or after a procedure such as angioplasty or stenting to reduce the level of restensosis in such subjects.

It is a still further object of the present invention to identify formulations useful for administration of one or more suitable drugs to a subject in need thereof, either before, during or after implantation of a drug loaded device (such as stent) to further reduce the level of restensosis over and above that which would have been achieved with the drug loaded device alone in such subjects.

It is yet another object of the present invention to identify formulations useful for administration of one or more drugs to a subject in need thereof to reduce the level of stensosis in such subjects that may at be at risk for stenosis of blood vessels.

These and other objects of the invention will become apparent upon inspection of the specification and claims provided herewith.

### SUMMARY OF THE INVENTION

In accordance with the present invention, there are provided methods for treating hyperplasia in a subject in need thereof. In another aspect of the invention, there are provided methods for reducing neointimal hyperplasia associated with vascular interventional procedures. In addition, there are provided formulations useful in this above-described methodsFormulations contemplated for use herein comprise proteins and at least one pharmaceutically active agent.

Invention formulations and methods offer the ability to develop drug delivery systems in a narrow size distribution with a mean diameter in the nanometer or micron size range (for comparison, a red blood cell is eight microns in diameter). Due to the particle size and composition, this delivery system allows for administration of the drug by various routes of delivery including intravenous, intraarterial, nasal, pulmonary, subcutaneous, intramuscular, oral and several other routes of administration.

Invention formulations provide several benefits over commercially available formulations of the same drugs. Some of these advantages include the fact that invention formulations are prepared employing biocompatible, non-toxic and well tolerated physiological protein components (e.g. human serum albumin) as excipients and stabilizers. Invention formulations are easily administered, for example, through angioplasty or stenting catheters, contain no toxic stabilizers, surfactants or solvents as vehicles in the formulations, and therefor present no danger of plasticizer leaching. Indeed, it has been demonstrated that invention compositions are readily amenable to parenteral administration by both intra-arterial and intravenous routes.

Invention formulations can be readily prepared as sterile filtered lyophilized formulations which are easily reconstituted with saline or dextrose. In addition, invention formulations display lower toxicity profiles with longer half-life of the active ingredient than do prior art formulations of the same active ingredient. Remarkably, generally no hypersensitivity reactions (usually attributable to toxic vehicles) are seen in patients, and no steroid premedication is required in patients to avoid hypersensitivity reactions. Invention formulations enable administration of higher dosing concentrations, which allow for small volume administration of the active agent. Doses of invention formulations can be administered by bolus I.V./ I.A. injection or over short infusion times (30 min or less). Morreover, standard infusion lines/bags (e.g., PVC) can be utilized for delivery of invention formulations as there is no plasticizer leaching due to absence of solvents and strong surfactants in invention formulations.

In accordance with the present invention, it has surprisingly been found that the combination of a biocompatible protein with drugs of interest greatly reduces the toxicity of such drugs when compared to commercially available preparations of the same drug.

In accordance with another aspect of the present invention, it has surprisingly been found that invention formulations, when administered systemically, can markedly reduce the level of restenosis following balloon angioplasty and stenting.

In accordance with yet another aspect of the present invention, it has surprisingly been found that invention compositions can markedly reduce the level of intimal hyperplasia or neointima formation following systemic administration of said compositions. This is contrary to the conventional wisdom that calls for coating of devices such as stents with the drug of interest and insertion or implantation of the device within the stenosed blood vessel in order to provide local delivery of the drug.

In accordance with still another aspect of the present invention, it has surprisingly been found that invention formulations may be administered at much higher doses and with substantially lower toxicity than commercially available formulations of the same drug.

In accordance with a still further aspect of the present invention, it has surprisingly been found that invention formulations may be administered intra-arterially without toxicity whereas commercially available formulations cannot be administered as such due to excessive toxicity.

In accordance with yet another aspect of the present invention, it has surprisingly been found that invention formulations may be delivered by inhalation for nasal or pulmonary absorption or by the oral route with excellent bioavailability whereas commercial preparations of similar drugs cannot be delivered by such routes of administration.

### BRIEF DESCRIPTION OF THE DRAWINGS

FIG. 1 shows the effect of varying paclitaxel concentrations on the proliferation of smooth muscle cells.
FIG. 2 shows the effect of varying paclitaxel concentrations on the migration of smooth muscle cells.

### DETAILED DESCRIPTION OF THE PREFERRED EMBODIMENTS

In accordance with the present invention, there are provided compositions useful for treatment of hyperplasia (e.g., when said hyperplasia occurs in blood vessel neointima), said compositions comprising at least one drug and protein.

In one aspect of the invention, said at least one drug is in nanoparticle form and is dispersed in said protein. Exemplary drugs contemplated for use herein include taxanes (e.g., paclitaxel) or analogs or homologs thereof, epothilones or analogs or homologs thereof, rapamycins or analogs or homologs thereof, and the like.

Invention formulations of the drugs of interest, for example, paclitaxel, rapamycin, steroids, etc. comprise biocompatible proteins, for example, albumin, casein, gelatin and the like.

Invention formulations can be administered systemically, e.g., intra-arterially, intravenously, by inhalation, orally, and the like, i.e., by any suitable means of delivery with minimal toxic side effects. Thus, for example, in the treatment of restenosis, the drug may be administered locally through the stenting cathether at the time of the procedure and at the local region of the stent. Invention formulations of the drug paclitaxel (also known as ABI-007 of Capxol), for example, afford the opportunity to administer paclitaxel at relatively high local concentration at the stent site with minimal systemic toxicity. ABI-007 may also be administered intravenously as support therapy to prevent restenosis. In addition, therapy with invention formulations may be provided by alternate routes of administration that are less invasive such as oral administration or by pulmonary or inhalational delivery.

Thus, for example, one of these invention formulations, ABI-007, a nanoparticle form of paclitaxel, has been extensively tested in human clinical studies for both intra-arterial and intravenous application with demonstration of efficacy, much lower toxicities and substantially higher MTD than the commercially available formulation of paclitaxel. To date, ABI-007 has been administered intra-arterially by percutaneous superselective arterial catheterization in over 120 patients and over 100 patients by intravenous administration.

In general, drugs that inhibit proliferation and migration of cells, e.g. antineoplastics (such as Taxanes, epthilones), antiproliferatives, immunosuppressives (e.g., cyclosporine, Tacrolimus, Rapamycin), peptide and protein drugs, angiogenesis inhibitors, and the like, are suitable candidates for invention compositions and methods of administration. An extensive list of suitable drugs is included in parent applications United States Application Serial No. 09/446,783 and PCT Application No. US98/13272, each of which is incorporated herein by reference in its entirety.

In accordance with another aspect of the present invention, there are provided compositions useful for reducing neointimal hyperplasia associated with vascular interventional procedure(s), said composition comprising at least one drug and protein. Compositions as described hereinabove are suitable for use in this aspect of the invention as well. As noted aove, such compositions can be delivered in a variety of ways, e.g., by systemic administration (e.g., intra-arterially, intravenously, by inhalation, orally, and the like).

Interventional procedures contemplated for use herein include angioplasty, stenting, atherectomy, and the like.

In accordance with another aspect of the present invention, there are provided pharmaceutical formulations with reduced toxicity, said formulations comprising a drug that inhibits proliferation and cell migration, and a biocompatible protein.

In accordance with still another aspect of the present invention, there are provided methods for treating hyperplasia in a subject in need thereof, said methods comprising administering to said subject an effective amount of a composition comprising drug and protein.

Presently preferred drugs employed in the practice of the present invention are in nanoparticle form and are dispersed in a suitable biocompatible protein.

As employed herein, "effective amount" refers to that amount of drug required to achieve the desired therapeutic effect. Generally, an effective amount will fall in the range of about 0.01 mg/kg up to about 15 mg/kg for a human subject. As readily recognized by those of skill in the art, active ingredient can be administered bolus, or over an extended period of time, for example, administration of said composition can be repeated over a dosing cycle between 1 day and 6 months.

Invention method can be carried out employing systemic administration (e.g., intra-arterially, intravenously, by inhalation, orally, and the like), and can be commenced before, during or after the occurrence of said hyperplasia.

In accordance with still another aspect of the present invention, there are provided methods for reducing neointimal hyperplasia associated with vascular interventional procedure(s) in a subject in need thereof, said methods comprising administering to said subject an effective amount of a composition comprising at least one drug and protein. Exemplary vascular interventional procedures contemplated for treatment herein include angioplasty, stenting, atherectomy, and the like. As readily recognized by those of skill in the art, invention compositions can be administered before, during or after the vascular interventional procedure.

In an alternate embodiment of the present invention, compositions contemplated for use herein can be administered at the time of the vascular interventional procedure. A particularly convenient way to accomplish this is to deploy a stent containing said at least one drug coated thereon.

As readily recognized by those of skill in the art, an effective amount of invention compositions is that amount which provides the desired therapeutic effect. Typically, effective amount will fall in the range of about 0.01 mg/kg up to about 15 mg/kg for a human subject. Administration can be conducted over a wide range of timeframes, typically being repeated from time to time, with intervals as short 1 day between doses, up to about 6 months or longer.

In accordance with yet another aspect of the present invention, there are provided methods to reduce the toxicity of a drug that inhibits proliferation and migration of cells, said method comprising combining said drug with a biocompatible protein.

Invention methods allow one to convert drugs such as paclitaxel, taxotere, taxanes and related compounds, epothilones and related compounds, rapamycin and related compounds, and the like, into nanoparticle formulations that can be easily administered by parenteral routes by utilizing biocompatible proteins, for example human serum albumin, which is non toxic and can be administered in large doses without problems in humans. Several nanoparticle formulations of various compounds have been prepared and tested *in vivo* with excellent safety profiles and efficacy. Invention formulations can be used to deliver therapeutic and pharmaceutic agents such as, but not limited to: antiproliferative/antimitotic agents including natural products such as vinca alkaloids (e.g., vinblastine, vincristine, and vinorelbine), paclitaxel, epidipodophyllotoxins (e.g., etoposide, teniposide), antibiotics (e.g., dactinomycin (actinomycin D) daunorubicin, doxorubicin and idarubicin), anthracyclines, mitoxantrone, bleomycins, plicamycin (e.g., mithramycin) and mitomycin, enzymes (e.g., L-asparaginase, which systemically metabolizes L-asparagine and deprives cells which don't have the capacity to synthesize their own asparagine); antiproliferative/antimitotic alkylating agents such as nitrogen mustards (e.g., mechlorethamine, cyclophosphamide and analogs, melphalan, chlorambucil), ethylenimines and methylmelamines (e.g., hexamethylmelamine and thiotepa), alkyl sulfonates-busulfan, nirtosoureas (e.g., carmustine (BCNU) and analogs, streptozocin),trazenes-dacarbazinine (DTIC); antiproliferative/antimitotic antimetabolites such as folic acid analogs (e.g., methotrexate), pyrimidine analogs (e.g., fluorouracil, floxuridine, and cytarabine), purine analogs and related inhibitors (e.g., mercaptopurine, thioguanine, pentostatin and 2-chlorodeoxyadenosine{cladribine}); platinum coordination complexes (e.g., cisplatin, carboplatin), procarbazine, hydroxyurea, mitotane, aminoglutethimide; hormones (e.g., estrogen); anticoaglants (e.g., heparin, synthetic heparin salts and other inhibitors of thrombin); fibrinolytic agents (such as tissue plasminogen activator, streptokinase and urokinase); antiplatelet (e.g., aspirin, dipyridamole, ticlopidine, clopidogrel, abciximab); antimigratory; antisecretory (e.g., breveldin); antiinflammatory: such as adrenocortical steroids (e.g., cortisol, cortisone, fludrocortisone, prednisone, prednisolone, 6.alpha.-methylprednisolone, triamcinolone, betamethasone, and dexamethasone), non-steroidal agents (e.g., salicylic acid derivatives, e.g., aspirin; para-aminophenol derivatives, e.g., acetominophen; indole and indene acetic acids (e.g., indomethacin, sulindac, and etodalac), heteroaryl acetic acids (e.g., tolmetin, diclofenac, and ketorolac), arylpropionic acids (e.g., ibuprofen and derivatives), anthranilic acids (e.g., mefenamic acid, and meclofenamic acid), enolic acids (e.g., piroxicam, tenoxicam, phenylbutazone, and oxyphenthatrazone), nabumetone, gold compounds (e.g., auranofin, aurothioglucose, gold sodium thiomalate); immunosuppressive: (e.g., cyclosporine, tacrolimus (FK-506), sirolimus (rapamycin), azathioprine, mycophenolate mofetil); Angiogenic: vascular endothelial growth factor (VEGF), fibroblast growth factor (FGF); nitric oxide donors; anti-sense olgio nucleotides and combinations thereof.

The invention will now be described in greater detail with reference to the following non-limiting examples.

### Example 1

### Effect of Paclitaxel Nanoparticles on Arterial Restenosis in Rats

Abnormal vascular smooth muscle proliferation (VSMP) is associated with cardiovascular disorders such as atherosclerosis, hypertension, and most endovascular procedures. Abnormal VSMP is a common complication of percutaneous transluminal coronary angioplasty (PTCA). The incidence of chronic restenosis resulting from VSMP following PTCA has been reported to be as high as 40-50% within 3-6 months.

The high incidence of vascular reocclusion associated with PTCA has led to development of in vivo animal model of restenosis and the search for agents to prevent it. The following study describes the use of Capxol™ in inhibiting restenosis following intimal trauma of the artery.

Male Sprague-Dawley Rats (Charles River) weighing 350-400 gm are anesthetized with Ketamin and Rompun and the right common carotid artery is exposed for a distance of 3.0 cm. The adherent tissue is cleared to allow two DIETRICH micro bulldog clamps to be placed about 2 cm apart around the carotid without causing crush injury to the vagus or associated superior cervical ganglion and sympathetic cord. No branches are present along this segment of the vessel. A 30-gauge needle attached to a 3 way stopcock is first inserted and then pulled out of the lower end of the isolated segment to make a hole on the wall of the vessel, and then inserted to the upper end for injection. 2-3 ml of phosphate-buffered saline is injected to rinse out all the blood inside the isolated segment then the 3-way stopcock is turned to another connection to a regulated source of compressed air. A gentle stream of air (25 ml per minute) is passed along the lumen of the vessel for 3 minutes to produce drying injury of the endothelium. The segment is then refilled with saline prior to removal of the needle from the vessel. Before the clamps are removed the needle holes on the vessel wall are carefully cauterized to prevent bleeding. A swab dampened with saline can be used to press on the needle holes to stop bleeding also. The skin is closed with 7.5-mm metal clips and washed with Betadine.

All the animals received the surgery described above and are sacrificed at the fourteenth day after surgery. The carotid artery on each side was retrieved for pathologic examination. The non-operated side serves as a self control. The experimental groups received different treatment as follows:
Group 1: High dose ABI-007 (Capxol™) treatment:
   paclitaxel 5 mg (w/ 100 mg Human Albumin)/kg/week, IV.
Group 2: Low dose ABI-007 (Capxol™) treatment:
   paclitaxel 1 mg (w/20 mg Human Albumin)/kg/week, IV.
Group 3: Drug vehicle control.
   Human Albumin 100 mg/kg/week IV.

The carotid artery biopsy samples are preserved in Formalin and then cross sections (8 µm) are cut from paraffin blocks and stained with hematoxylin and eosin. The cross-sectional areas of the blood vessel layers (intima, media, and adventitia) are quantified.

The injured carotid arteries in the control group showed remarkable accumulation of intimal smooth muscle cells and VSMC invasion of basement membrane. The overall thickness of the wall of carotid artery are doubled. The treatment groups showed a statistically significant decrease in the intimal wall thickening compared to the control.

### Example 2

### Systemic Delivery of Nanoparticle Paclitaxel (ABI-007) in a Rabbit Model of In-Stent Restenosis

This study was designed to examine a novel formulation of systemic paclitaxel (ABI-007, American BioScience, CA.) on in-stent restenosis in rabbit iliac arteries. Paciltaxel exerts its effect by preventing the depolymerization of microtubules. Although the antiproliferative effects of this drug are well documented, it has been known to delay healing in arterial injury models, especially with local delivery. It is thought that a systemic formulation of paclitaxel would allow steady control of drug levels and repeat dosing, potentially minimizing its effects on healing. To date, information on systemic delivery of paclitaxel in rabbits is limited, published toxicity studies have mostly been restricted to the rat. The study was conducted in three phases: 1) in-vitro assays of smooth muscle cell proliferation and migration (see Examples 3-5); 2) pharmacokinetics (see Example 6); and 3) in-stent restenosis (see Example 7).

### Example 3

### In-vitro tissue cultures to establish dose (inhibition of SMC proliferation & migration)

Smooth muscle cells (SMCs) isolated from the medial layer of the aorta from 3 male adult donor rabbits were cultured in M 199 supplemented with 10% Fetal Bovine Serum (FBS) and 100u/ml of penicillin and streptomycin. The cells were grown to confluence in 5% CO₂/95% air at 37° and used for proliferation and migration assays.

### Example 4

### Cell proliferation assay

SMC's (2×10⁴ cells per well) were seeded in 24-well culture plates and incubated with M-199 treated with 10% FBS in a humidified atmosphere of 5% CO₂/95% air. The next day, medium was changed and SMC's were further incubated for 48 hrs in M 199 and 1% FBS to synchronize the cells. SMC's were then stimulated in M199 treated with 10% FBS with and without various concentrations of paclitaxel. After 3 days of treatment, SMCs were trypsinized, and the number of cells counted using a hemocytometer. Analyses were done to include a battery of 2 different replicates using 2 different donors. The amount of SMC proliferation was expressed as a percentage of the control wells.

### Example 5

### Cell Migration Assay

Migration of SMC's was assayed in a 48-well chemotaxis chamber (Neuro Probe, Cabin John, MD). Briefly, cultured SMC's were trypsinized and suspended at a concentration of 5.0 × 10⁵ cells/ml in M-199 with 10% FBS. In the standard assay, a 50 µl volume of SMC suspension was placed in the upper chamber and 25 µl of M-199 containing a migration factor was placed in the lower chamber. Nanoparticle paclitaxel (1.0 nmol/L to 10 µmol/L American Bioscience, Santa Monica, CA), was added to both the upper and lower chambers at the same concentrations. Platelet derived growth factor (PDGF), added in the lower chamber at a concentration of 10 ng/ml, served as the chemoattractant. Assays were performed in which the total number of cells migrating through the gelatin coated polyvinylpyrrolidone-free polycarbonated membranes (8um pores; Nuclepore Corp., Pleasanton, CA) were quantified. Chambers were incubated at 37°C in a humidified atmosphere of 5% CO₂/95% air for 4 hours. After incubation nonmigrated cells in the upper chamber were wiped off gently. The filters were fixed in methanol and stained with Gill-3 hematoxylin (Shandon, Pittsburgh, PA). Migrated eels were counted using image analysis software (IP Lab spectrum, Signal Analytics Corp., Vienna, VA). Random migration was assessed by quantifying cell migration in response to medium alone. Analysis was done to include a battery of 2 different replicates using 2 different donors.

### Example 6

### In-vivo pharmacokinetics: Serum and local drug concentration at the stent site after systemic delivery

In this phase, stainless steel stents (ACS MULTI-LINK, Guidant Corp.) were deployed in both iliac arteries as described below; some arteries were balloon-injured without stenting. An intra-arterial infusion of radiolabelled [³H] paclitaxel nanoparticles (5 or 25 mg/kg, American BioScience CA.) was delivered at the time of stenting. These dosages were selected based on the findings of the in-vitro experiments (see Results). The drug was administered in a 10-ml volume over 5 minutes after the first stent was deployed through a catheter placed just proximal to the 1^{st} stent or balloon injury site. In cases with balloon-injury alone, [³H] paclitaxel was delivered after the first injury. Blood samples (1-ml) were taken immediately prior to stopping the infusion, 15 and 30 min, and 1, 3, 5, 8, 12, 24, and 48-hrs via a temporary jugular catheter. For each of the two dosing levels, three animals were used, one for stenting the other two for balloon-injury. After the study, tissue was harvested from the stent or balloon sites as well as control samples from the lung and liver. Radioactivity was quantified using a beta-counter to determine the local concentration of the drug, both at the site of delivery and the contralateral side.

### Example 7

### Suppression of In-Stent Restenosis by Systemic Paclitaxel

Several groups of rabbits (5 each) were treated with ABI-007 following balloon injury and stenting. They included a control arm that received no drug; a group receiving 1 mg/kg given on day 1; a group receiving 2.5 mg/kg given on day 1, a group receiving 3.5 mg/kg given on day 1; a group receiving 5 mg/kg given on day 1, a group receiving 15 mg/kg given on day 1; a group receiving 25 mg/kg given on day 1; and groups receiving the above doses repeated at intervals ranging between 1 day and 6 months.

All surgery was performed using aseptic techniques. Animals were premedicated with ketamine (100 mg IM) and buprenorphine (0.02 mg/kg IM) then anesthetized with isoflurane with 100% oxygen via facemask. Endotracheal intubation was performed, ventilation was initiated, and anesthesia was maintained with 3% isoflurane. Rabbits were placed in a supine position and the hindlegs abducted and externally rotated at the hips with the knees extended. A 5F sheath was inserted into the left common carotid artery exposed through a midline neck incision. Heparin (150 units/kg) was administered intra-arterially via the sheath. A 5F angiography catheter was placed in the distal aorta. Contrast dye (2 ml) was injected to obtain a control angiogram of the distal aorta and both iliac arteries. Iliac artery balloon injury was performed by inflating a 3.0 x 9.0 mm angioplasty balloon in the midportion of the artery followed by "pull-back" of the catheter for 1 balloon length. Balloon injury was repeated 2 times, and a 3.0 x 12 mm stent was deployed at 6 ATM for 30 seconds in the iliac artery. The rabbits were randomized to receive either paclitaxel or placebo. Immediately following stent placement, paclitaxel or normal saline was infused over a period of 5 minutes through the balloon catheter positioned just proximal to the stent. Balloon injury and stent placement was then performed on the contralateral iliac artery in the same manner described above. A post-stent deployment angiogram was performed. The proximal right carotid artery was ligated and the neck incision was closed in two layers. All animals received aspirin 40 mg/day orally and remained on a normal diet until euthanasia.

To assess cellular proliferation, animals received a subcutaneous injection of bromodeoxyuridine (BrdU, 100 mg/kg) and deoxycytidine (75 mg/kg) and an intramuscular injection of BrdU (30 mg/kg) and deoxycytidine (25 mg/kg) 18 hours prior to euthanasia. At 12 hours prior to euthanasia, they received an intramuscular injection of BrdU (30 mg/kg) and deoxycytidine (25 mg/kg).

### Example 8

### Euthanasia, Fixation, and Light Microscopy

Twenty-eight days after stenting, animals were anesthetized as above (ketamine IM, isoflurane via facemask and ventilation with 100% oxygen; anesthesia was maintained with inhaled isoflurane). A 5F sheath was placed in the right carotid artery, and a pre-euthanasia angiogram of the iliac arteries was performed. A 5F sheath was inserted into the jugular vein. Immediately prior to perfusion-fixation, rabbits received 1000 units of intravenous heparin. Euthanasia was accomplished with an injection of 1 ml of Beuthanasia given under deep anesthesia. The arterial tree was perfused at 100 mm Hg with lactated Ringer's until the perfusate from the jugular vein was clear of blood. The arterial tree was then perfused at 100 mm Hg with 10% formalin for 15 minutes. The distal aorta to the proximal femoral arteries was excised and cleaned of periadventitial tissue. Arteries were radiographed. The stents were embedded in plastic and sections were taken from the proximal, middle, and distal portions of each stent. All sections were stained with hematoxylin-eosin and Movat pentachrome stain. BrdU-positive cells were identified by established immunohistochemical techniques.

### Example 9

### Data Analysis

All arterial segments were examined with the observer blinded to the treatment group. Computerized planimetry was performed to determine the area of the IEL (internal elastic lamina), EEL (external elastic lamina), and lumen. The intima was measured at and between stent struts. The media and adventitia thickness were determined between stent struts. Percent luminal stenosis was calculated [1-(lumen/IEL)] ×100. To assess cellular proliferation, BrdU-positive cells in the intima and media were counted as a percentage of total cells (BrdU-labeling index) in 6 high power fields from the mid-segment of each stent. Data are expressed as the mean ± SEM. Statistical analysis of the histologic data was accomplished using analysis of variance (ANOVA). A p≤0.05 is considered statistically significant.

### Example 10

### Results of SMC Proliferation

Paclitaxel inhibited SMC proliferation in a dose dependent fashion. A statistically significant 55% inhibition was seen at 0.01uM concentration (p < 0.001) with a slight plateau in effect at higher doses (Table 1). The experiments were repeated in duplicate with two separate donors.

**Table 1**

| **Percentage Inhibition of SMC Proliferation on Day 3 With 72 Hour Exposure to Paclitaxel (ABI -007)** | | | | | |
|---|---|---|---|---|---|
| | Control | 0.001uM | 0.01uM | 0.1uM | 1uM |
| 9/7/00 | 0% | 21% | 61% | 53% | 61% |
| 10/19/00 | 0% | 28% | 48% | 61% | 59% |
| **Mean** | **0%** | **25%** | **55%** | **57%** | **60%** |
| SD | | 5% | 9% | 6% | 1% |
| P value | | P = NS | P < 0.001 | P < 0.001 | P < 0.001 |

The effect of paclitaxel on SMC proliferation was also studied after exposing the drug to SMC cultures for only 24 hours (Table 2). There was no real difference in the effect between the two groups.

**Table 2**

| **Percentage Inhibition of SMC Proliferation on Day 3 With 24 Hour Exposure to Paclitaxel (ABI -007)** | | | | | |
|---|---|---|---|---|---|
| | Control | 0.001uM | 0.01uM | 0.1uM | 1uM |
| 9/7/00 | 0% | 8% | 41% | 57% | 76% |
| 10/19/00 | 0% | 23% | 25% | 60% | 50% |
| **Mean** | **0%** | **16%** | **33%** | **59%** | **63%** |
| SD | | 11% | 11% | 2% | 18% |
| P value | | P = NS | P < 0.01 | P < 0.001 | P <0.001 |

### Example 11

### Results of SMC migration

Paclitaxel demonstrated profound inhibitory effects on SMC migration as tested in the chemotaxis chamber. At concentrations above 0.01uM paclitaxel showed significantly suppressed SMC migration (Table 3). The experiments were repeated in duplicates with two separate donors.

**Table 3**

| **Effect of ABI - 007 on Smooth Muscle Cell Migration in a 4-Hour Chemotaxis Assay using PDGF-BB as the Stimulant (% inhibition of control)** | | | | |
|---|---|---|---|---|
| | 0.001uM | 0.01uM | 0.1uM | 1uM |
| 9/7/00 | 24% | 53% | 62% | 84% |
| 10/19/00 | -7% | 15% | 80% | 92% |
| **Mean** | **9%** | **34%** | **71%** | **88%** |
| SD | 22% | 27% | 13% | 6% |
| P value | P=NS | P < 0.05 | P < 0.001 | P <0.0001 |

### Example 12

### Inhibition of Rat Smooth Muscle cell Proliferation and Migration

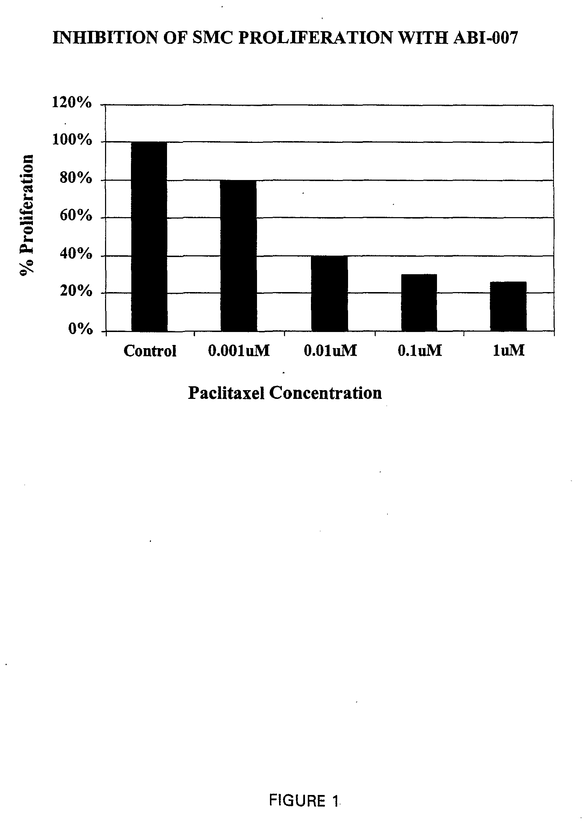

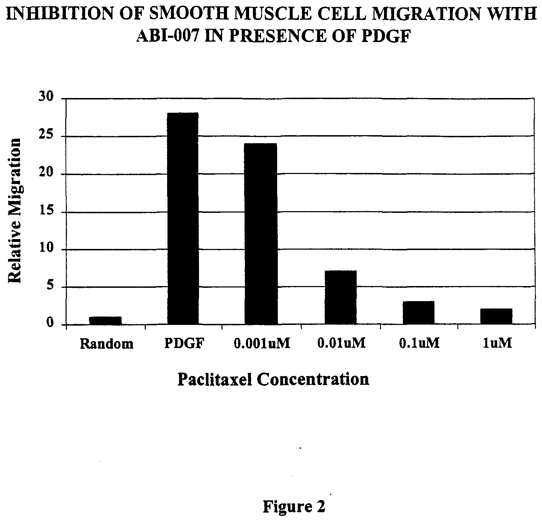

ABI-007 was also utilized to demonstrate inhibition of proliferation as well as migration in rat smooth muscle cells. The data in Figures 1 and 2 show the effect of varying paclitaxel concentrations on the proliferation and migration of smooth muscle cells. It is seen that at relatively low concentrations of 0.01 uM paclitaxel, ABI-007 is able to significantly inhibit the proliferative response (Figure 1) and migratory response (Figure 2) in rat.

### Example 13

### Results of Pharmacokinetic Studies

Pharmacokinetic studies were done in six rabbits, 3 with 25 mg/kg (rabbits A1, A2, A3) and 3 with 5 mg/kg (B1, B2, B3) with radiolabelled (tritiated) ABI-007 administered intrarterially immediately following the bilateral stenting of the iliac arteries. The blood levels showed a typical biphasic decline with an initial rapid decline followed by a slower elimination phase. Blood concentrations achieved for the 2 doses were substantially different as expected. At 12 hours post infusion, blood levels of ABI-007 as indicated by the radioactivity were approximately 0.8uM and 3uM for the 5mg/kg and 25mg/kg group respectively; at 24 hours these levels were approximately 0.5uM and 2.5uM repectively and at 48 hours these levels were approximately 0.4 and 2uM respectively. Thus, for at least 48 hours the blood levels of the compound were maintained significantly higher than the threshold of 0.01uM required for inhibition of proliferation and migration as determined by the in vitro experiments. The animals were euthanized at 24 (A1, A3, B1, B3) and 48 (A2, B2) hours.

### Example 14

### Determination of Local Tissue Concentrations of Paclitaxel

Local tissue concentration of radiolabelled paclitaxel was estimated after euthanizing the animals at time points described above (Table 4). The experiments were initially done with bilateral iliac artery stenting (A1, B1) and repeated in 4 additional animals with balloon denudation injury of both iliac arteries (A2, A3, B2, B3). There was no difference in paclitaxel concentrations between the right and the left iliacs despite exclusive infusion of the drug in the proximal right iliac artery.

**Table 4**

| **Local Paclitaxel Concentration (ug/gm of tissue) after Right Iliac Artery Infusion Proximal to the Injured Segment** | | | | | | | | | |
|---|---|---|---|---|---|---|---|---|---|
| No | Dose (mg/kg) | Injury type | Time estimated | Lt prox control | Lt Stent Site | Lt dist control | Rt Prox control | Rt Stent site | Rt dist control |
| A1 | 25 | Stent | 24 hrs | 3.9 | 2.8 | 3.7 | 4.0 | 3.6 | 5.0 |
| A2 | 25 | PTCA | 48 hrs | NA | 1.9 | 2.5 | 2.1 | 2.4 | 1.2 |
| A3 | 25 | PTCA | 24hrs | NA | 3.1 | 3.8 | 4.0 | 3.8 | 3.1 |
| B1 | 5 | Stent | 24 hrs | 1.8 | 1.6 | 1.5 | 2.5 | 1.2 | 2.1 |
| B2 | 5 | PTCA | 48 hrs | 0.9 | 0.8 | 1.1 | 1.0 | 0.5 | 1.4 |
| B3 | 5 | PTCA | 24 hrs | 4.5 | 1.3 | 1.6 | 2.7 | 1.5 | 1.5 |
| | | | | | | | | | |

### Example 15

### In vivo Studies in Rabbits

Technical Issues. Pre-stent balloon dilatation was evident by angiography. Bilateral iliac stent deployment in the rabbit was accomplished successfully in all cases. The stents were well deployed as visualized under fluoroscopy with contrast imaging. All arteries were widely patent at follow-up angiography 28 days after implant.

### Example 16

### Histologic Findings in Rabbit Studies

Despite balloon injury before stenting, disruption of the internal elastic lamina was uncommon in all groups (mean injury score <1). The neointima of control rabbits was well healed and consisted primarily of smooth muscle cells in a proteoglycan-rich matrix. Fibrin deposition around stent struts was rare. In rabbits treated with 5-mg/kg paclitaxel, there was evidence of delayed healing with fibrin deposition around stent struts, particularly remarkable in mid-sections. There was minimal endothelialization and inflammatory infiltrate. In the two rabbits that survived the 15-mg/kg dose, there was evidence of fibrin around and in-between stent wires in most sections. In some sections, the neointima consisted predominantly of fibrin with a few smooth muscle cells and acute inflammatory cells lining the lumen.

### Example 17

### Morphometric Analysis

A summary of the results of morphometric analysis is shown below in Table 5. When all sections (proximal, middle and distal) were included, there were signficant differences in some cases in mean intimal thickness, medial thickness, lumen area, neointimal area and percent stenosis in the 1, 2.5, 5 or 15 mg/kg paclitaxel groups versus controls. Similar findings were noted when comparing proximal, middle or distal sections.

**Table 5**

| **Summary of 28-day Morphometric Data (Values are expressed as mean ± SEM)** | | | |
|---|---|---|---|
| | Neointimal Thickness (mm) | Neointimal Area (mm²) | % Stenosis |
| Control | 0.128±0.01 | 1.58±0.07 | 25.9±1.1 |
| 1.0 mg/kg | 0.101±0.02 | 1.37±0.13 | 22.6±1.9 |
| 2.5 mg/kg | 0.098±0.01 | 1.31±0.03* | 22.4±0.61 |
| 5.0 mg/kg | 0.087±0.01* | 1.20+0.06** | 20.1±0.89* |
| 15.0 mg/kg | 0.078±0.01** | 1.10±0.13*** | 18.6±2.1** |
| p value vs. control | *0.002, **0.02 | *0.03, **<0.001, ***0.004 | *<0.001, **0.007 |

### Example 18

### Discussion of Results in the Rabbit Model of Restenosis

The potent effects of paclitaxel (ABI-007) on reducing smooth muscle proliferation and migration in-vitro were also apparent in our in-stent restenosis injury model. In animals receiving a single dose of paclitaxel ranging between 1 and 15-mg/kg, there was a significant increase in lumen area and a decrease in average neointimal thickness vs. control arteries. The decrease in intimal thickness with paclitaxel translated into a 13%-28% reduction in arterial stenosis. Cell proliferation in animals receiving 5 mg/kg and controls was <2% and was similar between groups; sections from the 15-mg/kg rabbits were not measured because of the acellular nature of the lesions and few number of cases. The paucity of proliferating cells is expected at 28 days after stenting although a persistence of cell proliferation has been identified with other treatments that delay healing such as radiation.

When the morphometric parameters from the proximal, middle, and distal regions of stent were averaged, there were marked differences among paclitaxel-treated and control animals; similar results were noted when only proximal and distal sections were compared.

Interestingly, there was a significant decrease in medial thickness in animals treated with 15-mg/kg paclitaxel. Typically, there is an acute reduction of medial smooth muscle cells after stenting, which recovers with time. These data suggest that paclitaxel may perhaps prevent the repopulation of smooth muscle cells after medial injury. It is also conceivable that the drug may be cytotoxic, particularly in cells that have been partially injured.

The concentration of the drug at the site of injury appears to be sufficient to suppress neointimal hyperplasia at 28 days. Transient exposure of paclitaxel (such as that achieved by systemic administration) may alter the microtubular function of the smooth muscle cells for sustained periods, impairing their mobility and proliferation. Repeat administration of invention formulations over preferred intervals of 1 week to 6 months will markedly improve long-term suppression of restenosis.

### Example 19

### Use of Systemic Administration in Combination with Drug-Loaded Stents

Slow release paclitaxel eluting stents (180 ug) have shown encouraging results up to 6 months in rabbit iliac arteries, however studies beyond this period are not available. Systemic administration of invention formulations in conjunction with drug loaded stents will improve long-term results in conjunction with local stent-delivery. Invention formulations for systemic delivery of desired drugs (e.g., paclitaxel and analogs, rapamycin and analogs, steroids, etc.) are contemplated to be utilized in conjunction with drug releasing devices such as stents to even further improve the suppression of restenosis after stenting or balloon injury.

### Example 20

### Dose ranges, Dosing Schedules and Repeat Dosing Studies

Optimal dose, dosing schedules, alternate routes of administration (e.g., intraarterial, intravenous, inhalation, oral, etc) were also investigated. For example, doses between 0.1 and about 30 mg/kg were investigated in rabbits and rats. Repeat dosing schedules, for example, initial dosing at the time of stenting or prior to stenting by any of the above modes of administration followed by repeat dosing by the above modes of administration at intervals ranging between 1 day to 6 months were possible. Dosing intervals of 1-6 weeks were especially preferred. The range of human doses covered were about 1mg/m² to about 375 mg/m². On a per kg basis in humans this would translate to about 0.05mg/kg-15mg/kg.

### Example 21

### In-vivo Preclinical Pharmacology and Toxicity Studies

The preclinical studies with ABI-007 were a combination of acute toxicity studies in mice; acute toxicity studies in rats; studies of myelosuppression in rats; pharmacokinetics studies in rats and an acute toxicity study in dogs. In most cases TAXOL was used as a comparator.

In a series of three pharmacokinetic studies in rats, the pharmacokinetic profile of paclitaxel, formulated as ABI-007, and TAXOL were shown to be similar, but blood/tissue concentration ratios and rates of metabolism varied significantly. ABI-007 is more rapidly distributed out of the blood and is more slowly metabolized. Tissue levels of radio-labeled paclitaxel were higher in several tissues (prostate, spleen, pancreas, and to a lesser extent bone, kidney, lung, and muscle) following administration of ABI-007 when compared to TAXOL. Excretion of paclitaxel following ABI-007 and TAXOL administration was predominantly in the feces.

Toxicity studies have been conducted in mice, rats, and dogs. Single dose acute toxicity studies in mice showed an LD₅₀ dose approximately 59 times greater for ABI-007 than for TAXOL. In a multiple dose toxicity study in mice, the LD₅₀ dose was approximately 10 fold greater for ABI-007 than for TAXOL.

In a 14 day, acute toxicity study in rats, the animals tolerated ABI-007 at doses up to 120 mg/kg, whereas significant morbidity and mortality were reported at doses of 30 mg/kg of TAXOL. Cerebral cortical necrosis, a serious toxic effect, was seen in the TAXOL-treated animals. Testicular degeneration was observed at higher doses in the ABI-007-treated animals.

### Example 22

### Human Clinical Data

ABI-007 has been studied in three separate Phase I human clinical trials, two by intravenous administration and another by intra-arterial administration. ABI-007 was well tolerated by patients upto doses of 300 mg/m² by both routes of administration. Pharmacokinetic data from both studies suggest that blood levels required to inhibit proliferation and migration of smooth muscle cells are easily achievable. The 0.01uM concentration of paclitaxel translates to 8.5 ng/ml. In Phase I clinical studies using both intra-arterial and intravenous administration of ABI-007, circulating blood levels of paclitaxel 24 hours after a short infusion (30 minutes) of ABI-007 remained close to or above 100 ng/ml. At 48 hours, blood levels were maintained above 10 ng/ml. This indicates that administration of ABI-007 either by the intra-arterial or intravenous route following angioplasty or stenting of a coronary artery can result in blood levels of the drug adequate to inhibit proliferation and migration of smooth muscle cells thus resulting in a positive outcome in restenosis of the injured blood vessel.

### Example 23

### Clinical Experience with ABI-007 - Intravenous Delivery

A phase I human clinical study of ABI-007 is complete. Nineteen patients were treated with ABI-007 administered by a 30 minute infusion every 21 days without the need for steroid premedication. The starting dose was 135 mg/m² escalated to 375 mg/m². 85 courses were administered and the maximum tolerated dose (MTD) was established at 300 mg/m². No hypersensitivity reactions were seen. No grade 3-4 hematologic toxicities were observed. No G-CSF support was given to any patient. The dose limiting toxicities were peripheral neuropathy and superficial keratitis.

A Phase II study of intravenous administration at a dose of 300mg/m² is ongoing. 50 Patients were dosed at 300 mg/m² by a 30 minute infusion every 21 days without the need for steroid premedication. The doses were well tolerated with acceptable toxicities. Another Phase II study of intravenous administration at a dose of 175mg/m² is ongoing. 40 Patients were dosed at 175 mg/m² by a 30 minute infusion every 21 days without the need for steroid premedication. The doses were well tolerated with acceptable toxicities.

### Example 24

### Clinical Experience with ABI-007 - Intra-arterial Delivery

A phase I human clinical study of ABI-007 given by intra-arterial injection has been completed. 100 patients were treated with ABI-007 administered by percutaneous superselective arterial catheterization of various arteries including but not limited to the carotid, femoral, hepatic, and mammary arteries in 30 minutes repeated every 4 weeks for 3 cycles. No steroid premedication was used. The dose was escalated from 125 mg/m² escalated to 300 mg/m². The maximum tolerated dose (MTD) was established at 270 mg/m². No hypersensitivity reactions were seen. No G-CSF support was given to any patient. The dose limiting toxicitiy was neutropenia. These data demonstrate the safety of intra-arterial administration of ABI-007.

### Example 25

### Other Drugs for Reduction of Neointima Formation

In general drugs that inhibit proliferation and migration of cells, e.g. Antineoplastics (such as Taxanes, epthilones), Antiproliferatives, Immunosuppressives (cyclosporine, Tacrolimus, Rapamycin), Peptide and protein drugs, angiogenesis inhibitors etare suitable candidates for administration by invention methods and formulations. An exhaustive list of drugs is included in WO99/00113 incorporated herein by reference in its entirety.

### Example 26

### Invention Compositions in Conjunction with Devices for delivery of Pharmacological Agents

Invention compositions, e.g., those containing drugs such as taxanes, are utilized in conjunction with devices for delivery in order to treat subjects in need of the medication or pharmaclogical agents. Devices comtemplated for use with invention compositions include but are not limited to any type of tubing including polymeric tubings that may be utilized to administer the invention compositions or in general to administer drugs such as the taxanes or other antiproliferative drugs. Tubings of interest for use in the invention include but are not limited to catheter of any type, intravenous lines, arterial lines, intra-thecal lines, intracranial lines, catheters or tubing that may be guided by suitable means to any location within the subject, e.g., to the site of a stenotic blood vessel such as coronary artery or other artery or vein. Such tubings may also have the capability to carry baloons or stents that are useful for treatment of local narrowing, stenosis, restenosis, plaques including atherosclerotic plaques, thrombotic lesions, sites of hyperplasia, aneurysms or weakness in blood vessels.

Devices such as stents are also contemplated as in combination with invention compositions. Stents may be fabricated from organic or inorganic materials, polymeric materials or metals. Invention compositions contemplate the combination of the invention pharmacological agents and devices mentioned herein.

Combination devices such as those comprising tubings along with baloons, stents, devices for local injection (e.g., into the lumen, into the vessel wall, into the intima of the blood vessel, into the endothelial or sub-endothelial layer, into the smooth muscle layer of blood vessels) etc. are also contemplated in combination with invention compositions of pharmacological agents.

Invention compositions of pharmacological agents or in general drugs such as the taxanes or other antiproliferative drugs and any drug or drugs contemplated by the invention may be delivered by the devices described above either by flowing through the device, being impregnated or embedded or stored within or with the device, or being able to be released or delivered at a local site of interest by the device or delivered by the device to be systemically available in the subject (e.g., intravenous administration).

### Example 27

### Pulmonary Delivery of ABI-007 (paclitaxel)

The purpose of this study was to determine the time course of [³H]ABI-007 in blood and select tissues following intratracheal instillation to Sprague Dawley rats. The target volume of the intratracheal dose formulation to be administered to the animals was calculated based on a dose volume of 1.5 mL per kg body. The dosing apparatus consisted of a Penn-Century microsprayer (Model 1A-1B; Penn-Century, Inc., Philadelphia, PA purchased from DeLong Distributors, Long Branch, NJ) attached to a 1-mL gas-tight, luer-lock syringe. The appropriate volume of dose preparation was drawn into the dosing apparatus, the filled apparatus was weighed and the weight-recorded. A catheter was placed in the trachea of the anesthetized animal, the microsprayer portion of the dosing apparatus was placed into the trachea through the catheter, and the dose was administered. After dose administration the empty dosing apparatus was reweighed and the administered dose was calculated as the difference in the weights of the dosing apparatus before and after dosing. The average dose for all animals was 4.7738 ± 0.0060 (CV 1.5059) mg paclitaxel per kg body weight.

Blood samples of approximately 250 µL were collected from the indwelling jugular cannulas of JVC rats at the following predetermined post-dosing time points: 1, 5, 10, 15, 30, and 45 min and 1, 4, 8, and 24 h. The 24-h blood samples, as well as blood samples collected from animals sacrificed at 10 min, 45 min, and 2 h, were collected via cardiac puncture from anesthetized rats at sacrifice. All blood samples analyzed for total radioactivity were dispensed into pre-weighed sample tubes, and the sample tubes were reweighed, and the weight of each sample was calculated by subtraction. The blood samples collected from the jugular vein as well as ca. 250-µL aliquots of blood collected from each animal at sacrifice were assayed for total tritium content (see Table 6).

**TABLE 6**

| Noncompartmental analysis of blood tritium concentration (mg-eq/L) vs. time profiles in rats after intratracheal instillation of [³H]ABI-007 | | |
|---|---|---|
| Parameter | Mean ± SD | |
| Cₘₐₓ (mg-eq/L) | 1.615 | ± 0.279 |
| Tₘₐₓ (hr) | 0.0833 | ± 0.0 |
| t_{1/2β} (hr) | 33.02 | ± 11.99 |
| AUCₗₐₛₜ (mg-eq × hr/L) | 7.051 | ± 1.535 |
| Cl/F (L/hr) | 0.0442 | ± 0.0070 |
| F^{a} (Bioavailability) | 1.229 | ± 0.268 |

For all rats, the maximum concentration of tritium in blood was observed at 5 min (0.0833 hr) post dosing. The elimination half-life of tritium, determined over the time interval from 4 hr to 24 hr, ranged from 19.73 hr to 43.02 hr. It should be noted that this interval includes only three data points, which may account for the variability in this parameter. The apparent clearance of tritium from blood was on the order of 0.04 L/hr.

The mean blood concentration of [³H]ABI-007-derived radioactivity after an intravenous dose to rats was analyzed as a function of time in order to evaluate the bioavailability of tritium derived from an intratracheal dose of [³H]ABI-007. This analysis resulted in a 24-hour AUC (AUCₗₐₛₜ) of 6.1354 mg-eq × hr/L. Based on these data, radioactivity derived from the intratracheal dose of [³H]ABI-007 is highly bioavailable. These analyses are based on total radioactivity.

Tritium derived from [³H]ABI-007 is rapidly absorbed after intratracheal instillation. The average absorption and elimination half-lives (k₀₁ half-life and k₁₀ half-life, respectively) for tritium in blood after an intratracheal dose of [3H]ABI-007 (mean ± SD) were 0.0155 ± 0.0058 hr and 4.738 ± 0.366 hr, respectively. The average apparent clearance of tritium from blood was 0.1235 ± 0.0180 L/hr.

Tritium derived from [³H]ABI-007 was absorbed and distributed after intratracheal administration. The time course of tritium in blood was well described by a two-compartment model, with mean absorption and elimination half-lives of 0.0155 and 4.738 hr, respectively. Approximately 28% of the administered dose was recovered in the lung at 10 min after the intratracheal dose. A maximum of less than 1% of the dose was recovered in other tissues, excluding the gastrointestinal tract, at all time points examined.

Based on results from a previously conducted intravenous dose study with [³H]Capxol™, the bioavailability of tritium derived from the intratracheal dose was 1.229 ± 0.268 (mean ± SD) for the three animals in this dose group. It should be noted, however, that this estimate of bioavailability is based on total radioactivity and may therefore not be indicative of the true bioavailability of paclitaxel.

A fair amount of radioactivity was present in the gastrointestinal tract (including contents) at 24 hr post dosing (27% for the intratracheal dose). The presence of tritium in the gastrointestinal tract may be due to biliary excretion or clearance of tritium from the respiratory tract via mucociliary clearance with subsequent swallowing.

### Example 28

### Oral Delivery of ABI-007 (paclitaxel)

Tritiated ABI-007 was utilized to determine oral bioavailablity of pqaclitaxel following oral gavage in rats. Following overnight fasting 5 rats were given 5.5 mg/kg paclitaxel in ABI-007 (Group A) and another 5 rats (Group B) were pretreated with cyclosporin (5.0 mg/kg) followed by 5.6 mg/kg paclitaxel in ABI-007. A pharmacokinetic analysis of blood samples drawn at 0.5, 1, 2, 3, 4, 5, 6, 8, 12, and 24 hours was performed after determination of radioactivity in the blood samples by combustion. Oral biovailability was determined by comparison with intravenous data previously obtained. The results are tabulated in Table 7 below.

**Table 7**

| **Mean AUC ₀₋₂₄, Cmax, Tmax and % Absorption of ³H-Paclitaxel Derived Radioactivity Following Oral Administration** | | | | | | |
|---|---|---|---|---|---|---|
| Group | Treatment | Dose/Route (mg/kg) | AUC₀₋₂₄ (µg eq x hr/mL) | Absorption (%) | Cₘₐₓ (µg x eq/mL) | Tₘₐₓ (hr) |
| A | ABI-007 in Normal Saline | 5.5/PO(P) | 2.92 | 44.3 | 0.245 | 1 |
| B | ABI-007 in Normal Saline with CsA | 5/PO(C), 5.6/PO(P) | 8.02 | 121.1 | 0.565 | 0.5 |

| | | | | | | |
|---|---|---|---|---|---|---|
| Note: AUC₀₋₂₄ IV (6.06 µg x hr./mL) and IV dose (5.1 mg/kg) have been used for calculation of percent absorption, data based on IV dose of ABI-007. | | | | | | |

An oral bioavailability of 44% was seen for ABI-007 alone. This is dramatically higher than is seen for other formulations of paclitaxel. The biovailability increased to 121% when animals were treated with cyclosporine (CsA). This is expected as CsA is a known suppressor of the p-glycoprotein pump that would normally prevent absorption of compounds such as paclitaxel from GI tract. The greater than 100% bioavailability can be explained by reabsorption following biliary excretion of paclitaxel into the GI tract. Other known suppressors or enhancers of absorption may be also utilized for this purpose.

While the invention has been described in detail with reference to certain preferred embodiments thereof, it will be understood that modifications and variations are within the spirit and scope of that which is described and claimed.

The application discloses inter alia following items:
1. A method for treating hyperplasia in a subject in need thereof, said method comprising administering to said subject an effective amount of a composition comprising drug and protein.
2. A method according to item 1 wherein said drug is in nanoparticle form and is dispersed in said protein.
3. A method according to item 1 wherein said hyperplasia occurs in blood vessel neointima.
4. A method according to item 1 wherein said effective amount falls in the range of about 0.01 mg/kg up to about 15 mg/kg for a human subject.
5. A method according to item 4 wherein said administration of said composition is repeated over a dosing cycle between 1 day and 6 months.
6. A method according to item 1 wherein said composition is administered systemically.
7. A method according to item 6 wherein administration is accomplished intra-arterially, intravenously, by inhalation, or orally.
8. A method according to item 1 wherein said composition is administered before, during or after the occurrence of said hyperplasia.
9. A method for reducing neointimal hyperplasia associated with vascular interventional procedure(s) in a subject in need thereof, said method comprising administering to said subject an effective amount of a composition comprising at least one drug and protein.
10. A method according to item 9 wherein said procedure comprises angioplasty, stenting or atherectomy.
11. A method according to item 9 wherein said composition is administered before, during or after the vascular interventional procedure.
12. A method according to item 9 wherein said composition is administered at the time of the vascular interventional procedure.
13. A method according to item 9 wherein said effective amount falls in the range of about 0.01 mg/kg up to about 15 mg/kg for a human subject.
14. A method according to item 13 wherein said administration of said composition is repeated over a dosing cycle between 1 day and 6 months.
15. A method according to item 9 wherein said composition is administered systemically.
16. A method according to item 9 wherein said composition is administered by deployment of a stent containing said at least one drug coated thereon.
17. A method to reduce proliferation and cell migration in a subject undergoing a vascular interventional procedure, said method comprising systemically administering a formulation comprising a drug that inhibits proliferation and cell migration, and a biocompatible protein to said subject before, during or after said procedure.
18. A composition for treatment of hyperplasia, said composition comprising at least one drug and protein.
19. A composition according to item 18 wherein said at least one drug is in nanoparticle form and is dispersed in said protein.
20. A composition according to item 18 wherein said hyperplasia occurs in blood vessel neointima.
21. A composition according to item 18 wherein said drug is a taxane or analog or homolog thereof, an epothilone or analog or homolog thereof, or a rapamycin or analog or homolog thereof.
22. A composition according to item 21 wherein said taxane is paclitaxel.
23. A composition according to item 18 wherein said composition is suitable for systemic administration.
24. A composition according to item 23 wherein administration is accomplished intra-arterially, intravenously, by inhalation, or orally.
25. A composition for reducing neointimal hyperplasia associated with vascular interventional procedure(s), said composition comprising at least one drug and protein.
26. A composition according to item 25 wherein said procedure is angioplasty, stenting or atherectomy.
27. A composition according to item 25 wherein said composition is suitable for systemic administration.
28. A composition according to item 27 wherein administration is accomplished intra-arterially, intravenously, by inhalation, or orally.
29. A method to reduce the toxicity of a drug that inhibits proliferation and migration of cells, said method comprising combining said drug with a biocompatible protein.
30. A pharmaceutical formulation with reduced toxicity, said formulation comprising a drug that inhibits proliferation and cell migration, and a biocompatible protein.

## Claims

1. A pharmaceutical composition comprising nanoparticles comprising rapamycin and human serum albumin.

2. The pharmaceutical composition of claim 1, wherein said rapamycin is dispersed in said human serum albumin.

3. A pharmaceutical composition as defined in claim 1 or 2 for use in a method of treating hyperplasia in a subject in need thereof.

4. The pharmaceutical composition for use of claim 3, wherein said hyperplasia is hyperplasia of blood vessel neointima.

5. The pharmaceutical composition for use of claim 3 or 4, wherein said composition is administered systemically.

6. The pharmaceutical composition for use of claim 5, wherein said composition is administered intraarterially, intravenously, by inhalation, or orally.

7. The pharmaceutical composition for use of claim 6, wherein said composition is administered intraarterially by bolus injection to an artery.

8. The pharmaceutical composition for use of any one of claims 3 to 7, wherein said hyperplasia of blood vessel neointima is restenosis.

9. The pharmaceutical composition for use of any one of claims 3 to 8, wherein said hyperplasia is associated with vascular interventional procedure.

10. The pharmaceutical composition for use of claim 9, wherein said vascular interventional procedure is angioplasty, stenting or atherectomy.

11. The pharmaceutical composition for use of any one of claims 3 to 10, wherein said subject is a human.

12. The pharmaceutical composition for use of claim 11, wherein said rapamycin is administered in an amount of about 0.01 mg/kg up to about 15 mg/kg.
